# EUROPEAN PATENT APPLICATION

(11) **EP 2 626 341 A1**
(43) Date of publication of application: **14.08.2013**
(21) Application number: 12154817.6
(22) Date of filing: 10.02.2012
(51) Int. Cl.: C07C 17/18, C07C 29/124, C07C 31/38, C07C 19/12, C07F 7/18, C07C 309/64, C07C 309/66, C07C 309/73

(54) **Manufacture of hexafluoroisopropanol**

(71) Applicant: SOLVAY SA, 1120 Bruxelles (BE)
(72) Inventor: Braun, Max Josef, 30900 WEDEMARK (DE); Kasubke, Michael, 30173 Hannover (DE)
(74) Representative: Mross, Stefan P.M.

(57) **Abstract**

Hexafluoroisopropanol (CF₃CHOHCF₃) is manufactured in two steps from monochloromalonic acid or monochlomalonic acid esters by a reaction with a fluorinating agent, notably SF₄, to form CF₃CHClCF₃ which is hydrolyzed to form CF₃CHOHCF3.

The hexafluoroisopropanol can be used as such, or, preferably, it is further reacted to form Sevoflurane, an anesthetic.

Compounds of the formula CF₃-CH(OY)-CF₃ wherein Y is OTs, OMe, OTf or OTMS are also described.

## Description

The present invention concerns a method for the manufacture of 1,1,1,3,3,3-hexafluoropropanol.

1,1,1,3,3,3-hexafluoropropanol, also denoted as "hexafluoro-isopropanol" or "HFIP", is a versatile compound, used especially to solubilize peptides, and as solvent for certain polymers, for example, polyamides, polyacrylonitriles, polyacetals, polyesters (e.g. polyglycolides), and polyketones which are not dissolved easily by other solvents. It also is an intermediate in the manufacture of Sevoflurane, an inhalation anesthetic. WO-A- 2010/125899 discloses the manufacture of Sevoflurane by reacting hexafluoroisopropanol with bis(fluoromethyl)ether in the presence of a strong acid.

A well-known way for its manufacture comprises the hydrogenation of hexafluoroacetone.

Object of the present invention is to provide an efficient process for the manufacture of 1,1,1,3,3,3-hexafluoropropanol. This object and other objects apparent from the description and the claims is achieved by the present invention.

The process of the present invention concerns the manufacture of 1,1,1,3,3,3-hexafluoropropanol comprising
a) a first step of the manufacture of 2-chloro-1,1,1,3,3,3-hexafluoropropane by reacting chloromalonic acid or a chloromalonic acid ester with a fluorinating agent having the formula (I), R-SF₃, wherein R is F or R¹R²N, wherein R¹ and R² are the same or different an denote C1 to C3 alkyl ; C1 to C3 alkyl substituted by a methoxy group, under formation of chloro-1,1,1,3,3,3-hexafluoropropane, and
b) a second step wherein chloro-1,1,1,3,3,3-hexafluoropropane is hydrolyzed to form 1,1,1,3,3,3-hexafluoropropanol.

Preferably, R is F or R¹R²N wherein R¹ and R² are the same and denote methyl, ethyl or 2-methoxyethyl. Consequently, the most preferred fluorinating agents are SF₄, diethylaminosulfur trifluoride (DAST), dimethylaminosulfur trifluoride (DMAST) and bis (2-methoxyethyl)aminosulfur trifluoride (Deoxofluor).

SF₄ is especially preferred as fluorinating agent.

The production of SF₄ can be realized according to the procedure described in the GB Pat. No. 1374054.

According to one embodiment, the fluorination with SF₄ is carried out in the presence of anhydrous HF. HF is often used in an amount of about 1 to about 1000 moles, and preferably about 1 to about 500 moles, per mole of SF₄.

In the process according to the present invention, the fluorination can be carried out in the presence of a solvent. The solvent to be used may also, for example, be an aromatic hydrocarbon such as benzene, toluene or xylene, an aliphatic hydrocarbon such as pentane or hexane ; a halogenated hydrocarbon such as methylene chloride, chloroform, ethylene dichloride, hydrofluorocarbons, e.g. 1,1,1,3,3-pentafluorobutane (Solkane^{®}365 mfc) or perfluorocarbons, e.g. perfluorocyclohexane ; or an ether such as diethyl ether, dibutyl ether or tetrahydrofuran. Among them, the halogenated hydrocarbons are preferred. Methylene chloride is most preferred. These solvents may be used alone or in combination as a mixture. If appropriate, the solvent is used usually in an amount of from 50 to 99 % by weight, preferably from 60 to 99 % by weight, more preferably from 75 to 99 % by weight of the solvent relative to the total weight of the reaction medium.

If the reaction mixture is a liquid under the reaction conditions, a solvent is not needed. If desired, starting compounds or reaction products could be used as solvent. Particularly preferred is the use of Sevoflurane as solvent. Preferably, if Sevoflurane is used as a solvent, it is present in the reaction mixture from the start.

The pressure and the temperature are selected as such that the reaction mixture remains in the liquid phase.

The process according to the present invention generally comprises carrying out the fluorination at a temperature equal to or higher than 30°C, preferably equal to or higher than 40°, more preferably equal to or higher than 50°C. It is generally carried out at a temperature equal to or lower than 90°C, preferably at a temperature equal to or lower than 80°C, more preferably a temperature equal to or lower than 70°C. A temperature ranging from 50 to 70°C is most preferred.

The process according to the present invention generally comprises carrying out the fluorination under pressure. The pressure of the fluorination is advantageously equal to or greater than about 5 bar (abs), preferably about 10 bar (abs) to equal to or less than about 25 bar (abs).

Stoichiometrically, the transformation of 1 C(O) group to a CF₂ group consumes one molecule of SF₄. The transformation of a C-OR ester group (in case, a monochloromalonic monoester or monochloromalonic diester is used as a starting material) to a C-F group consumes half a molecule of SF₄, as well as the transformation of a OCH₂X group to form a OCH₂F group. Consequently, in the process according to the invention, the range of the molar ratio of substituted malonic acid derivative to SF₄ preferably is from 1:3 to 1:5, and particularly preferably from 1:3,5 to 1:4.5.

If desired, the fluorination can be carried out in 2 or even 3 steps. In the 1^{st} step, any hydroxyl groups are transformed. In a second step, the other groups, e.g. the carbonyl groups, are transformed. The first step often is performed at a lower temperature range, e.g. at - 80°C to 0°C. The second step is preferably performed at the temperature given above.

The boiling point of chlorohexafluoropropane is supposed to be -4°C. It can be isolated from the reaction mixture for example by pressure distillation or by fractionated condensation.

The chlorohexafluoropropane formed in step a) is then hydrolyzed to form 1,1,1,3,3,3-hexafluoropropanol.

The substitution by the C1 atom can be achieved by a reaction with potassium acetate and subsequent acidic hydrolysis.

The hexafluoroisopropanol formed has a boiling point of 58.2°C and can be isolated from the reaction mixture by known methods, e.g. by distillation.

As mentioned above, hexafluoroisopropanol can be applied as such, especially as a solvent for polymers, e.g. polyamides, and for peptides.

In a preferred embodiment, the hexafluoropropanol is subjected to one or more further reaction steps to provide Sevofluorane which is CF₃-CH(OCH₂F)-CF₃.

The transformation of CF₃-CHOH-CF₃ to Sevoflurane can be performed in a known manner.

According to a first embodiment, CF₃-CHOH-CF₃ is reacted with trioxane or formaldehyde and HF as described in US-A 6,100,434.

According to a second embodiment, an intermediate compound is manufactured having the formula CF₃-CH(OCH₂X)-CF₃ which is fluorinated. X is OH or a leaving group which can be substituted by nucleophilic substitution and which is preferably selected from halogens or oxygen containing functional groups such as OTos and OTMS. X is preferably selected from bromine, chlorine, iodine, OH, OTos or OTMS ; more preferably X is chlorine or OH, most preferably X is chlorine or fluorine.

In general, the fluorination reaction is performed in the liquid phase.

In the process according to the invention, the fluorination is generally carried out with fluorinating agents. Said fluorinating agents preferably allow fluorinating a carbon-halogen group to form carbon-fluorine groups. Typical examples of said fluorinating agents are for example SF₄ (sulfur tetrafluoride) ; diethylaminosulfur trifluoride (DAST), dimethylaminosulfur trifluoride (DMAST) and bis (2-methoxyethyl)aminosulfur trifluoride (Deoxofluor).

SF₄ is most preferred as fluorinating agent. The production of SF₄ can be realized according to the procedure described in GB Pat. No. 1374054.

According to one embodiment, the fluorination with SF₄ is carried out in the presence of anhydrous HF. HF is often used in an amount of about 1 to about 1000 moles, and preferably about 1 to about 500 moles, per mole of SF₄.

In the process according to the present invention, the fluorination can be carried out in the presence of a solvent. The solvent to be used may also, for example, be an aromatic hydrocarbon such as benzene, toluene or xylene, an aliphatic hydrocarbon such as pentane or hexane ; a halogenated hydrocarbon such as methylene chloride, chloroform, ethylene dichloride, hydrofluorocarbons, e.g. 1,1,1,3,3-pentafluorobutane (Solkane^{®}365 mfc) or perfluorocarbons, e.g. perfluorocyclohexane ; or an ether such as diethyl ether, dibutyl ether or tetrahydrofuran. Among them, the halogenated hydrocarbons are preferred. Methylene chloride is most preferred. These solvents may be used alone or in combination as a mixture. If appropriate, the solvent is used usually in an amount of from 50 to 99 % by weight, preferably from 60 to 99 % by weight, more preferably from 75 to 99 % by weight of the solvent relative to the total weight of the reaction medium.

If the reaction mixture is a liquid under the reaction conditions, a solvent is not needed. If desired, starting compounds or reaction products could be used as solvent. Particularly preferred is the use of Sevoflurane as solvent. Preferably, if Sevoflurane is used as a solvent, it is present in the reaction mixture from the start.

The pressure and the temperature are selected as such that the reaction mixture remains in the liquid phase.

The process according to the present invention generally comprises carrying out the fluorination at a temperature equal to or higher than 30°C, preferably equal to or higher than 40°, more preferably equal to or higher than 50°C. It is generally carried out at a temperature equal to or lower than 90°C, preferably at a temperature equal to or lower than 80°C, more preferably a temperature equal to or lower than 70°C. A temperature ranging from 50 to 70°C is most preferred.

The process according to the present invention generally comprises carrying out the fluorination under pressure. The pressure of the fluorination is advantageously equal to or greater than about 5 bar (abs), preferably about 10 bar (abs) to equal to or less than about 25 bar (abs).

The transformation of OCH₂X group to form a OCH₂F group consumes half a molecule of SF₄. Consequently, in this embodiment, the range of the molar ratio of substituted malonic acid derivative to SF₄ preferably is from 1:0.5 to 1:1.

The compounds having the formula CF₃-CH(OCH₂X)-CF₃ wherein X is OH or a leaving group which can be substituted by nucleophilic substitution, can principally be manufactured in two alternatives : according to one embodiment, the synthesis is performed with an O-nucleophile, and according to another embodiment, the synthesis is performed with a CH₂-electrophile.

Thus, it is possible to manufacture the compounds of formula CF₃-CH(OCH₂X)-CF₃ wherein X is OH or a leaving group which can be substituted by nucleophilic substitution, said leaving group preferably being selected from halogens or oxygen containing functional groups such as OTos and OTMS by reaction of a compound having formula CF₃-CH(OCH₂Y)-CF₃ wherein Y is a leaving group which can be substituted by nucleophilic substitution. Y is preferably selected from halogens or oxygen containing functional groups such as OTos and OTMS, with an O-nucleophile.

Typical examples of O-nucleophiles are formaldehyde and formaldehyde derivatives.

Formaldehyde and formaldehyde derivatives are preferred O-nucleophiles.

For the purpose of the present invention, the term "formaldehyde derivative" refers to a class of molecules which are formed from formaldehyde by polymerization, trimerisation or addition reactions such as paraformaldehyde, trioxane, hydrated formaldehyde, hemiacetalic structures of formaldehyde of formula HO-CH₂-X wherein X is a leaving group which can be substituted by nucleophilic substitution and which is preferably selected from halogens or oxygen containing functional groups. Typical examples of said hemiacetalic compounds are for instance HO-CH₂-OTos and HO-CH₂OTMS.

In general, the paraformaldehyde or trioxane is cracked, e.g. thermally or chemically, to form the monomeric formaldehyde.

The process according to the present invention generally comprises carrying out the reaction with the O-nucleophile in the presence of a solvent. The solvent to be used may, for example, be a polar solvent such as dimethyl formamide, dimethyl acetamide, dimethylsulfoxide, sulfolane, Sevoflurane, tetrahydrofuran, acetonitrile, dioxane, a halogenated hydrocarbon such as methylene chloride, chloroform or ethylene dichloride ; or an ether such as diethyl ether, dibutyl ether or tetrahydrofuran. Among them, a polar solvent is preferred. Particularly preferred among them, is dimethyl formamide, sulfolane or Sevoflurane. These solvents may be used alone or in combination as a mixture. If appropriate, the solvent is used usually in an amount of from 50 to 99 by weight, preferably from 60 to 99 % by weight, more preferably from 75 to 99 % by weight of the solvent relative to the total weight of the reaction medium.

If desired, the reaction with the O-nucleophile optionally may be carried out in the presence of a base. If a base is used, it may be an inorganic base or an organic base. The inorganic base may be selected from the group consisting of alkali metal hydroxides such as sodium hydroxide, potassium hydroxide, lithium hydroxide, cesium hydroxide, alkaline earth metal hydroxides such as calcium hydroxide, barium hydroxide, magnesium hydroxide, strontium hydroxide, and basic alkali metal salts such as sodium carbonate, sodium hydrogencarbonate, potassium carbonate and potassium hydrogencarbonate. Preferred bases are sodium hydroxide and potassium hydroxide. Most preferred base is sodium hydroxide . The organic base may be selected from the group consisting of nitrogen-containing heterocyclic compounds such as pyridine, quinoline or picoline ; and tertiary bases such as triethylamine, dimethylaniline, diethylaniline and 4-dimethylaminopyridine. Among them, pyridine, triethylamine, dimethylaniline, diethylaniline and 4-dimethylaminopyridine are preferred. Among them, pyridine is particularly preferred. These bases may be used alone or in combination as a mixture.

If desired, the base can be applied in the form of an aqueous solution or a solution of the base in water and an organic solvent. The organic solvent is in particular a solvent as described above.

For the purpose of the present invention, the term "aqueous solution" refers to solutions of water soluble compounds in water, for example salt water or any other aqueous mineral salt solution.

In the process according to the invention, the molar ratio of the base to O-nucleophile is advantageously from 0.5:1 to 2:1, preferably from 0.7:1 to 1.5:1, and more preferably from 0.8:1 to 1.2:1. Most preferably, the molar ratio is about 1.

Further details on the transformation of OH groups or OX groups, as described above, and fluorination can be found in WO 2011/018466.

According to another alternative of the second embodiment, it is possible to manufacture the compounds of formula CF₃-CH(OCH₂X)-CF₃ wherein X is OH or a leaving group which can be substituted by nucleophilic substitution, said leaving group preferably being selected from halogens or oxygen containing functional groups such as OTos and OTMS, by the reaction of a compound having formula CF₃-CH(OH)-CF₃ with a CH₂-electrophile.

Formaldehyde is most preferred CH₂-electrophile.

Optionally, the hydroxyl derivative F₃C-CH(OCH₂OH)-CF₃ can be converted to the substituted malonic acid derivative of formula F₃C-CH(OCH₂X)-CF₃ wherein X is in particular a leaving group, as described above. The conversion can be realized by reaction of said hydroxyl substituted compound with PCl₃, PCl₅, TosOH, MesOH or amine·nHCl like Py·HCl.

According to the most preferred alternative of the second embodiment, the compounds having the formula F₃C-CH(OCH₂X)-CF₃ wherein X is a leaving group which can be substituted by nucleophilic substitution. Said leaving group is preferably selected from halogens or oxygen containing functional groups such as OTos and OTMS are reacted with a CH₂-electrophile in the presence of a leaving group transfer agent.

For the purpose of the present invention, the term "leaving group transfer agent" refers to a class of compounds that are able to transfer their leaving group to another compound. Typical examples of leaving group transfer agents are for example a chlorinating agent selected from the group consisting of oxalyl chloride, phosphorus trichloride, phosphorus pentachloride or phosphorus oxychloride, a thionyl compound such as thionyl chloride and thionyl fluoride ; a sulfuryl compound such as sulfuryl chloride and sulfuryl fluoride ; a tosylating agent such as p-toluenesulfonyl chloride (tosyl chloride) and p-toluenesulfonyl fluoride (tosyl fluoride), or a mesylating agent, such as methanesulfonyl chloride (mesyl chloride) and methanesulfonyl fluoride (mesyl fluoride).

Preferred leaving group transfer agents are thionyl and sulfuryl compounds. Most preferred leaving group transfer agents are thionyl compounds. Preferred thionyl compounds are thionyl chloride and thionyl fluoride.

In a particular aspect of this embodiment, the reaction of the hydroxyl compound having the formulaF₃C-CH(OH)-CF₃ with a CH₂-electrophile in the presence of a leaving group transfer agent may be carried out additionally in the presence of a strong acid. The strong acid may be selected for example from the group consisting of fuming sulfuric acid, fuming nitric acid and hydrogen halides such as in particular hydrogen chloride, hydrogen fluoride.

In a first specific way, the strong acid is a fuming sulfuric acid.

In a second specific way, the strong acid is hydrogen chloride or hydrogen fluoride.

In a preferred aspect, X is chlorine or fluorine, and the compounds of formula (I) are F₃C-CH (OCH₂Cl)-CF₃ and F₃C-CH (OCH₂F)-CF₃ (Sevoflurane). F₃C-CH (OCH₂Cl)-CF₃ can be fluorinated to form Sevoflurane as described above.

According to an improved mode, CF₃-CHOH-CF₃ is reacted with at least one CH₂-electrophile selected from the group consisting of (a) bis(fluoromethyl)ether (CH₂FOCH₂F) and (b) formaldehyde activated with HF and at least one additional activator. Further details to this type of reaction can be found in unpublished EP patent application 11194988.9 filed on December 21, 2011 in the name of Solvay SA.

In general, the reaction is performed in the liquid phase.

In the process according to this improved mode, the pressure and the temperature are therefore usually selected as such that the reaction mixture remains in the liquid phase.

The process according to the present invention generally comprises carrying out the reaction at a temperature equal to or higher than 0°C, preferably equal to or higher than 40°, more preferably equal to or higher than 50°C. It is generally carried out at a temperature equal to or lower than 150°C, preferably at a temperature equal to or lower than 120°C, more preferably a temperature equal to or lower than 100°C. A temperature ranging from 50 to 70°C is most preferred.

In the process according to the invention, the molar ratio between formaldehyde or bis(fluoromethyl)ether and CF₃-CHOH-CF₃ is generally from 1,0 to 5,0, preferably from 1,1 to 2,0.

In one aspect, the process according to the present invention comprises carrying out the reaction in the presence of a solvent, in particular an aprotic organic solvent. The solvent to be used may, for example, be a polar solvent such as an amide type solvent, in particular dimethyl formamide or dimethyl acetamide, anitrile, in particular acetonitrile, a sulphur containing solvent, in particular dimethylsulfoxide or sulfolane, a halogenated hydrocarbon such as methylene chloride, chloroform or ethylene dichloride ; or an ether such as diethyl ether, dibutyl ether, dioxane or tetrahydrofuran.

Fluorinated solvents, for example a fluorinated hydrocarbon such as in particular 1,1,1,3,3-pentafluorobutane, or a fluorinated ether such as Sevoflurane are preferred. Particularly preferred solvents are selected from dimethyl formamide, sulfolane, 1,1,1,3,3-pentafluorobutane or Sevoflurane. These solvents may be used alone or in combination as a mixture. If appropriate, the solvent is used usually in an amount of from 50 to 99 by weight, preferably from 60 to 99 % by weight, more preferably from 75 to 99 % by weight of the solvent relative to the total weight of the reaction medium.

When the CH₂-electrophile is bis(fluoromethyl)ether, the reaction is preferably carried out in the presence of at least one additional activator.

When an additional activator is used in the process according to the invention, it is generally a strong acid other than HF. The strong acid may be selected for example from the group consisting of sulfuric acid, in particular fuming sulfuric acid, nitric acid, in particular fuming nitric acid and hydrogen chloride. In another aspect, the strong acid is a perfluoroalkylsulphonic acid, such as in particular trifluoromethylsulphonic acid or p-trifluoromethylbenzenesulphonic acid. The additional activator is preferably sulphuric acid. In the process according to the invention, the molar ratio between additional activator and formaldehyde or bis(fluoromethyl)ether is generally from 0,001 to 0,5, preferably from 0,002 to 0,01.

When the CH₂-electrophile is formaldehyde activated with HF and at least one additional activator, the molar ratio HF/formaldehyde is generally from 1 to 10, preferably from 2 to 5.

According to another embodiment, the CF₃-CHOH-CF₃ is reacted with an O-nucleophile to provide an intermediate compound of formula (I), CF₃-OCH₂X-CF₃ wherein X is a leaving group, preferably Cl. The intermediate compound of formula (I) wherein X is Cl, can be manufactured as described in US patent 6,100,434 by reaction of hexafluoroisopropanol and trioxane or formaldehyde in the presence of a catalyst, especially AlCl₃.

If desired, CF₃-CHOH-CF₃ is, in a preliminary step, reacted to provide CF₃-CHOY-CF₃ wherein Y is a leaving group, for example, OTos or OTMS [OTMS denotes O-Si(CH₃)₃]. This type of transformation may easily be conducted by reaction of the hydroxyl group with respectively p-toluenesulfonic acid, methanesulfonic acid, trifluoromethanesulfonic acid, trimethylsilyl chloride or bis(trimethylsilyl)acetamide. It is also possible to replace the hydroxyl group of CF₃-CHOH-CF₃ with a halogen other than fluorine, in particular bromine, chlorine or iodine, more particularly chlorine. Such reactions are detailed in international patent application WO 2011/018466 which is incorporated herein by reference in its entirety.

The formed intermediate compound CF₃-CH(OCH₂X)-CF₃ or CF₃-CH(OCH₂Y)-CF₃ can then be reacted to form the desired compound CF₃-CH(OCH₂F)-CF₃.

This can expediently be performed by using a fluorinating agent.

In a specific third embodiment, compounds having the formula CF₃-CH(OCH₂X)-CF₃ wherein X is a leaving group that can be substituted by nucleophilic substitution, a reaction can be performed with at least one source of nucleophilic fluorine to obtain the desired compound CF₃-CH(OCH₂F)-CF₃. More information about this type of reaction can be found in unpublished international patent application PCT/EP2012/052035, filed February 7, 2012 in the name of Solvay SA.

In the process of the present invention, the source of nucleophilic fluorine is preferably a fluoride, more preferably a fluoride selected from the group consisting of inorganic fluorides and organic fluorides, especially from metal fluorides and organic fluorides, e.g. onium fluorides, more particularly from alkali fluorides and ammonium fluorides, most preferably from alkali fluorides. Suitable examples of metal fluorides are silver(I)fluoride (AgF) and silver(II)fluoride (AgF₂). Suitable examples of alkali fluorides are sodium fluoride (NaF), potassium fluoride (KF), lithium fluoride (LiF), and cesium fluoride (CsF), advantageously potassium fluoride. Examples of ammonium fluorides are NH₄F and R₄NF where R is an alkyl group such as methyl, ethyl, propyl or butyl.

In this third embodiment, the substituent X in compounds having the formula CF₃-CH(OCH₂X)-CF₃ may typically be selected from the group consisting of halogens except fluorine ; and oxygen containing functional groups, preferably from bromine, chlorine, iodine, O-tosylate (OTos), O-mesylate (OMes), O-triflate (OTf) and O-trimethylsilyl (OTMS), more preferably from chlorine, OTos, OMes and OTMS, especially preferably chlorine.

The process of the third embodiment is typically conducted in liquid phase. This process is usually conducted in the presence of a solvent, preferably a polar solvent, more preferably a polar aprotic solvent. Examples of suitable solvents are alkyl nitriles or alkylene dinitriles, e.g. acetonitrile, dialkylsulfoxides, e.g. dimethylsulfoxide (DMSO), dialkylsulfones, e.g. dimethylsulfone (DMSO₂) cyclic sulfones, e.g. sulfolane, formamides, e.g. dimethylformamide (DMF), pyrrolidones, e.g. N-methyl-2-pyrrolidone (NMP), ketones, e.g. acetone, esters, e.g. ethyl acetate, cyclic ethers, e.g. tetrahydrofurane, halogenated carbons which optionally may also comprise hydrogen as substituent or substituents, e.g. dichloromethane, aromatic compounds, e.g. toluene, fluorosubstituted aromatic compounds, e.g. CF₃-toluene, and ionic liquids, e.g. those ionic liquids described in US patent application publication 2009/0242840. In this paragraph, the term "alkyl" preferably denotes methyl, ethyl, n-propyl or isopropyl ; the term "alkylene" preferably denotes methylene, ethylene or propylene.

The fluoride in the source of nucleophilic fluorine is usually used in at least a stoechiometric amount, in particular with a molar ratio of fluoride to compounds of formula (III) or (IV) from 4:1 to 1:1, more particularly from 3:1 to 1:1, especially from 2:1 1 to 1:1, for example around 1.5.

The reaction according to the third embodiment is in general conducted under atmospheric pressure. The temperature is usually from 40 to 200°C, often from 50 to 150°C, more often from 70 to 160°C. Most often, the reaction is conducted near the reflux temperature, which will thus depend on the nature of the solvent used.

The duration of the reaction of the process of the third embodiment is typically from 15 minutes to 24 hours, in many cases from 30 minutes to 12 hours, for instance from 1 to 8 hours.

In the process of the third embodiment, the reaction may be performed in an acid, an acidic, neutral or basic medium, preferably in a neutral or basic medium, more preferably in a neutral medium. The medium may be acidified, neutralized or basified by addition of any suitable pH modifying agent known in the art. Acidic pH modifying agents are for example hydrochloric acid, nitric acid, phosphoric acid, sulfuric acid, formic acid, acetic acid, ascorbic acid, citric acid, or tartaric acid. Alkaline pH modifying agents are for example sodium hydroxide, potassium hydroxide, soda ash, ammonia, or triethylamine. The starting material of formula (III) or (IV) may also be acidified, neutralized or basified prior to its use in the process of the present invention, preferably neutralized or basified, more preferably neutralized, using the same pH modifying agents as described above. The starting material may then further be washed, in particular with water or with an organic solvent, and dried prior to its use in the process of the present invention.

According to a variation of the third embodiment, the process of the present invention may be carried out in the presence of at least one alkali halide other than alkali fluorides, for instance in the presence of an alkali iodide, such as potassium iodide (KI) or cesium iodide (CsI), and preferably in the presence of potassium iodide. The addition of at least one other alkali halide and in particular of an alkali iodide, especially potassium iodide, has the advantage to accelerate the reaction. Indeed, alkali iodides such as KI or CsI dissolve better than KF and dissociate faster (easier) than KF, thus starting the reaction. Such additional alkali halides are usually used in an amount of about 0.01 to 0.5 mol per mol of fluoride, in many cases from 0.05 to 0.2 mol per mol of fluoride, for instance around 0.1 mol per mol of fluoride.

If an intermediate compound is used as starting material wherein Y is OMes, OTf, OTos or OTMS, a fluorinating agent having the formula (I), R-SF₃ can be applied. Here, R has the meaning given above ; SF₄ is the preferred fluorinating agent.

The purification of the Sevoflurane obtained can be performed as described in WO 2009/085247 by washing it several times with water.

Another aspect of the present invention concerns intermediate compounds having the formula CF₃-OCH₂Y-CF₃ wherein Y is selected from OTos, OMes, OTf, and OTMS. OTos denotes the p-toluenesulfonate anion, OMes denotes the methanesulfonate anion, OTf denotes the trifluoromethanesulfonate anion, and OTMS denotes the anion (CH₃)₃SiO⁻. The manufacture of these compounds can be performed from hexafluoroisopropanol and respective tosylating, mesylating, triflating and trimethylsilylating agents, e.g. as is described above. They can be used as intermediates for the manufacture of Sevoflurane.

The conversion of the hexafluoroisopropanol can be performed in solvents, e.g. in CCl₄ CHCl₃, acetonitrile or dimethylformamide.

Should the disclosure of any patents, patent applications, and publications which are incorporated herein by reference conflict with the description of the present application to the extent that it may render a term unclear, the present description shall take precedence.

The examples here after are intended to illustrate the invention without however limiting it.

### Example 1 : Manufacture of CF₃-CHOH-CF₃ from CF₃-CHCl-CF₃

A suspension of 63g (642 mmol, 2.5 equivalents) of potassium acetate in 250 ml of dimethyl formamide is given into a reaction vessel. Under nitrogen, 257 mmol of CF₃-CHCl-CF₃ are introduced. The reaction mixture is stirred overnight at room temperature and mixed with water and a saturated aqueous NaCl solution. After drying over Na₂SO₄, the supernatant liquid is filtrated off, concentrated at reduced pressure, dissolved in ethanol and mixed with approximately 3 ml of concentrated H₂SO₄. After stirring overnight, the reaction mixture is concentrated under vacuum, and mixed with water and ethyl acetate. The organic phase is washed with water until the pH is neutral, and then dried over Na₂SO₄. Hexafluoroisopropanol is distilled out of the residue (boiling point 58.2°C).

### Example 2 : Synthesis of F₃C-CH(OCH₂F)-CF₃ by reaction of F₃C-CH(OH)-CF₃ with bis(fluoromethyl ether)

A solution of 0,3 moles bis-fluoromethylether in 25 ml 1,1,1,3,3-pentafluorobutane is prepared and 0,1 mole F₃C-CH(OH)-CF₃, dissolved in 25 ml 1,1,1,3,3-pentafluorobutane is added at 20°C. Stirring is continued for 1h. The product F₃C-CH(OCH₂F)-CF₃ is isolated by distillation.

### Example 3 : Synthesis of F₃C-CH(OCH₂F)-CF₃ by reaction of F₃C-CH(OH)-CF₃ with formaldehyde/HF/H₂SO₄

A solution of 0,3 moles bis-fluoromethylether in 25 ml methylene chloride is prepared by dissolving 0.3 moles paraformaldehyde in 25ml methylene chloride and addition of 1,5 moles anhydrous HF dissolved in 25 ml 1,1,1,3,3-pentafluorobutane under ice cooling. To this solution, 0,1 mole F₃C-CH(OH)-CF₃, dissolved in 25 ml 1,1,1,3,3-pentafluorobutane is added at 20°C. Finally, 0,01 mole of H₂SO₄ diluted in 1 mL 1,1,1,3,3-pentafluorobutane is added dropwise. Stirring is continued for 1h. The product F₃C-CH(OCH₂F)-CF₃ is isolated by distillation.

### Example 4 : Manufacture of CF₃-CH(OTMS)-CF₃

Bis-(trimethylsilyl)acetamide is commercially available. Alternatively, it can be manufactured as described by J. F. Klebe et al. In JACS 88 (1966), pages 3390 - 3395, "Experimental Section", example 1, from acetamide and trimethylsilyl chloride in the presence of triethyl amine.

Hexafluoroisopropanol (46.7g, 0.25 mol) and bis-trimethylsilylamide (20 % molar excess) are dissolved, under nitrogen, in the same volume of CHCl₃. After 10 minutes, CHCl₃ was removed under vacuum, and the residual reaction mixture was distilled to provide CF₃-CH(OTMS)-CF₃.

## Claims

1. A method for the manufacture of 1,1,1,3,3,3-hexafluoropropanol comprising
a) a first step of the manufacture of 2-chloro-1,1,1,3,3,3-hexafluoropropane by reacting chloromalonic acid or a chloromalonic acid ester with a fluorinating agent having the formula (I), R-SF₃, wherein R is F or R¹R²N, wherein R¹ and R² are the same or different an denote C1 to C3 alkyl ; C1 to C3 alkyl substituted by a methoxy group, under formation of chloro-1,1,1,3,3,3-hexafluoropropane, and
b) a second step wherein chloro-1,1,1,3,3,3-hexafluoropropane is hydrolyzed to form 1,1,1,3,3,3-hexafluoropropanol.

2. The method of claim 1 wherein the fluorinating agent is SF₄.

3. The method of claim 1 or 2 the range of the molar ratio of chloromalonic acid or chloromalonic acid ester to SF₄ is from 1:3 to 1:5.

4. The method of anyone of claims 1 to 3 wherein the fluorination is performed at a temperature equal to or higher than 30°C.

5. The method of anyone of claims 1 to 4 wherein the fluorination is performed at a temperature equal to or lower than 90°C.

6. The method of anyone of claims 1 to 5 wherein the pressure during the fluorination is equal to or greater than about 5 bar (abs).

7. The method of anyone of claims 1 to 6 wherein the pressure during the fluorination is equal to or less than about 25 bar (abs).

8. The method of anyone of claims 1 to 7 wherein the hydrolysis of step b) is performed using potassium acetate and subsequent acidification.

9. The method of anyone of claims 1 to 8 wherein the hydrolysis is performed at a temperature equal to or greater than 0°C.

10. The method of anyone of claims 1 to 8 wherein the hydrolysis is performed at a temperature equal to or lower than 100°C.

11. The method of anyone of claims 1 to 10 further comprising a subsequent step of reacting the 1,1,1,3,3,3-hexafluoropropanol formed to produce Sevoflurane, CF₃-CH(OCH₂F)-CF₃.

12. The method of claim 11 further comprising a step of reacting the 1,1,1,3,3,3-hexafluoropropanol formed with a CH₂-electrophile, preferably formaldehyde or trioxane, and HF to produce Sevoflurane, CF₃-CH(OCH₂F)-CF₃.

13. The method of claim 11 further comprising a step of reacting the 1,1,1,3,3,3-hexafluoropropanol formed with a CH₂F-electrophile, preferably bis(fluoromethyl)ether, to produce Sevoflurane, CF₃-CH(OCH₂F)-CF₃.

14. The method of claim 11 further comprising a step of reacting the 1,1,1,3,3,3-hexafluoropropanol formed with a CH₂-electrophile, preferably trioxane or formaldehyde, in the presence of AlCl₃ to produce an intermediate compound having the formula CF₃-OCH₂Cl-CF₃ and subsequent chlorine-fluorine exchange.

15. The method of claim 11 further comprising a step of reacting the 1,1,1,3,3,3-hexafluoropropanol formed with p-toluenesulfonic acid, methanesulfonic acid, trifluoromethanesulfonic acid, trimethylsylil chloride or bis-trimethylsilyl)acetamide to produce an intermediate compound having the formula CF₃-OCH₂Y-CF₃ wherein Y is a leaving group selected from OTos, OMes, OTf, and OTMS, and a subsequent step of reacting the intermediate compound of formula CF₃-OCH₂Y-CF₃ with a fluorinating agent to produce Sevoflurane, CF₃-CH(OCH₂F)-CF₃.

16. Compounds having the formula CF₃-OCH₂Y-CF₃ wherein Y is selected from OTos, OMes, OTf, and OTMS.
